# EUROPEAN PATENT SPECIFICATION

(11) **EP 1 507 009 B1**
(45) Date of publication and mention of the grant of the patent: **18.11.2009**
(21) Application number: 03752935.1
(22) Date of filing: 21.05.2003
(51) Int. Cl.: C12N 15/82, C12N 15/33

(54) **VECTOR FOR GENE SILENCING AND GENE SILENCING METHOD USING THE SAME**
GEN-SILENCING-VEKTOR UND DIESEN VERWENDENDES GEN-SILENCING-VERFAHREN
VECTEUR DE SILENAGE GENIQUE ET PROCEDE DE SILEN AGE GENIQUE UTILISANT CE VECTEUR

(30) Priority: 22.05.2002 JP 2002147888
(43) Date of publication of application: 16.02.2005
(73) Proprietor: Japan Tobacco Inc., Tokyo 105-8422 (JP)
(72) Inventor: Kasaoka, Keisuke, Japan Tobacco Inc., Oyama-shi, Tochigi 323-0808 (JP); Saito, Yasuhito, Japan Tobacco Inc., Oyama-shi, Tochigi 323-0808 (JP); Kuwata, Shigeru, Lab. of Plant Biotechnology, Kawasaki-shi (JP)
(74) Representative: Behnisch, Werner
(86) International application number: PCT/JP2003/006328
(87) International publication number: WO 2003/097841

(56) References cited:
- WO-A-98/36083
- WO-A-03/050288
- JP-A- 6 133 783
- RUIZ, M.T., ET AL.: "initiation and maintenance of virus-induced gene silencing" THE PLANT CELL, vol. 10, 1998, pages 937-946, XP002248311
- KUMAGAI M H ET AL: "CYTOPLASMIC INHIBITION OF CAROTENOID BIOSYNTHESIS WITH VIRUS-DERIVED RNA" PROCEEDINGS OF THE NATIONAL ACADEMY OF SCIENCES OF USA, NATIONAL ACADEMY OF SCIENCE, WASHINGTON, DC, US, vol. 92, 1 February 1995 (1995-02-01), pages 1679-1683, XP002012921 ISSN: 0027-8424
- BURTON R A ET AL: "Virus-induced silencing of a plant cellulose synthase gene" PLANT CELL, AMERICAN SOCIETY OF PLANT PHYSIOLOGISTS, ROCKVILLE, MD, US, vol. 12, no. 5, May 2000 (2000-05), pages 691-705, XP002335669 ISSN: 1040-4651
- ENGLISH J ET AL: "Suppression of virus accumulation in transgenic plants exhibiting silencing of nuclear genes" PLANT CELL, AMERICAN SOCIETY OF PLANT PHYSIOLOGISTS, ROCKVILLE, MD, US, vol. 8, no. 2, February 1996 (1996-02), pages 179-188, XP002095871 ISSN: 1040-4651
- GUO H S ET AL: "Plum pox potyvirus resistance associated to transgene silencing that can be stabilized after different number of plant generations" GENE: AN INTERNATIONAL JOURNAL ON GENES AND GENOMES, ELSEVIER, AMSTERDAM, NL, vol. 206, no. 2, 12 January 1998 (1998-01-12), pages 263-272, XP004108904 ISSN: 0378-1119
- VOINNET O: "RNA silencing as a plant immune system against viruses" TRENDS IN GENETICS, ELSEVIER, AMSTERDAM, NL, vol. 17, no. 8, 1 August 2001 (2001-08-01), pages 449-459, XP004296190 ISSN: 0168-9525
- VANCE VICKI ET AL: "RNA silencing in plants: Defense and counterdefense" SCIENCE, AMERICAN ASSOCIATION FOR THE ADVANCEMENT OF SCIENCE,, US, vol. 292, no. 5525, 22 June 2001 (2001-06-22), pages 2277-2280, XP002311148 ISSN: 0036-8075
- BAULCOMBE D C: "RNA AS A TARGET AND AN INITIATOR OF POST-TRANSCRIPTIONAL GENE SILENCING IN TRANSGENIC PLANTS" PLANT MOLECULAR BIOLOGY, SPRINGER, DORDRECHT, NL, vol. 32, no. 1/2, 1996, pages 79-88, XP009004417 ISSN: 0167-4412
- ANGELL S.M. ET AL.: 'Consistent gene silencing in transgenic plants expressing a replicating potato virus X RNA' EMBO J. vol. 16, no. 12, 1997, pages 3675 - 3684, XP002067897
- SONODAL S. ET AL.: 'Delayed activation of post-transcriptional gene silencing and de novo transgene methylation in plants with the coat protein gene of sweet potato feathery mottle potyvirus' PLANT SCI. vol. 156, no. 2, 2000, pages 137 - 144, XP002970018
- BRIGNETI G. ET AL.: 'Viral pathogenicity determinants are suppressors of transgene silencing in Nicotiana benthamiana' EMBO J. vol. 17, no. 22, 1998, pages 6739 - 6746, XP002174427
- QU F. ET AL.: 'The coat protein of turnip crinkle virus suppresses post transcriptional gene silencing at an early initiation step' J. VIROL. vol. 77, no. 1, January 2003, pages 511 - 522, XP002970019

## Description

### Technical Field

The present invention relates to the use of a virus vector for causing gene silencing (referred to as a gene silencing vector hereinafter) and to a gene silencing method using the vector. More particularly, the present invention relates to a gene silencing method of virus-induced type that exhibits a high occurrence probability of gene silencing and that is inherited according to the Mendel's laws.

### Background Art

Suppression of the expression of a specific target gene is a promising technique not only for an aspect of investigation for the purpose of analysis of gene functions but also for plant breeding. As a means for that purpose, there is known an antisense method, which introduces an antisense DNA having a base sequence whose direction is opposite to the direction of the target gene into a cell to inhibit the translation of the target gene. However, the antisense method has the problem that complete inhibition of the production of protein is difficult.

To solve the above-mentioned problem involved in the antisense method, studies of gene silencing have recently been stepped up. Gene silencings are classified into one that acts on the level of transcription of a gene (Transcriptional Gene Silencing; TGS) and one that acts after transcription (Post Transcriptional Gene Silencing; PTGS). Both of them differ from the antisense method in that in most cases, suppression of endogenous gene expression close to a range of 100% occurs frequently. Recently, there have been many research reports on PTGS; the phenomenon of rapid decomposition of transcribed mRNA is reported in mammals, plants and microorganisms (SCIENCE vol. 288, 1370-1372; NATURE vol. 404, 804-808; etc.) Although the mechanism of PTGS is at present still unknown, the phenomenon is known as Co-Suppression in the field of plant biotechnology.

However, the problem has been pointed out that the co-suppression exhibits a low occurrence probability of PTGS, and that it is frequently observed for the induction of PTGS to occur only in a portion of the tissue.

Further, virus-induced gene silencing (VIGS) that uses a virus vector in order to induce PTGS efficiently is known. However, VIGS has the problems that it involves use of virus per se as a vector and that gene silencing is not inherited.

The inventors of the present invention have previously disclosed in JP 6-133783 A, a vector for providing potato cells with resistance to potato virus Y necrosis strain (PVY-N). Subsequent studies have led to the finding that the vector can be used for gene silencing and completion of the present invention. That is, the present invention is providing the use of a gene silencing vector that suppresses the expression of specific target gene in a host .

### Disclosure of Invention

A gene silencing vector used according to the present invention is a recombinant vector that comprises an enhancer sequence downstream of a promoter and a gene encoding a coat protein of potyvirus-origin (hereinafter, occasionally referred to as "CP-encoding gene") downstream of the CP-coding gene, wherein a specific target gene construct which comprises a promoter, a sequence of the target gene and a terminator is inserted downstream of the terminator of the vector.

Further, the gene silencing method according to the present invention comprises transforming a host plant with a gene construct obtained by inserting the target gene into the vector, thereby suppressing expression of the target gene in the host plant.

The gene silencing vector according to the present invention or the gene silencing method according to the present invention bring about such unexpected effects that:
(1) high occurrence probability of gene silencing can be achieved even when there is only one copy of the target gene;
(2) individuals can be provided in which expression of the target gene is controlled at various levels (suppressed in a range of several% to 100%) in the present generation;
(3) gene silencing can be inherited in the progeny; and
(4) varieties can be easily attained in which expression of the target gene is suppressed in a range of several% to 100% by crossing with a fixed variety, because the gene silencing is inherited conforming to the Mendel's laws.

### Brief Description of Drawings

FIG. 1 is a diagram illustrating construction of PVY-T/CPW vector.
FIG. 2 is a diagram illustrating construction of pYS415 vector.
FIG. 3 is a diagram illustrating construction of pYS412 vector.
FIG. 4 is a diagram illustrating construction of pYS436 vector.
FIGS. 5A and 5B are diagrams illustrating construction of pYS465 vector.
FIG. 6 is a diagram illustrating construction of pYS466 vector.

In those diagrams, symbols therein are as follows:
NPT = neimycin phosphotransferase, BR = right border, BL =left border, 35s-pro = cauliflower mosaic virus 35s promoter, NOS-ter = terminator of nopaline synthetase, Amp = ampicillin resistant gene, PVY-CP = coat protein gene of PVY-T, GFP = GFP gene, GUS = β-glucuronidase gene, and PsDof1 = pea zinc flinger transcription factors.

### Best Mode for Carrying Out the Invention

Hereinafter, the present invention will be described in detail.

The inventors of the present invention noted that, in a GS-PVY vector (JP 6-133783 A) developed in order to make potato resistant to potato virus Y necrosis line, a gene incorporated around a PVY-T coat protein in a sense direction is induced to cause gene silencing in a host, and found that an enhancer-encoding gene and a coat protein-encoding gene of virus-origin both of which exist in the downstream of a promoter relate to PTGS deeply.

The GS-PVY vector is a recombinant vector that has a leader sequence of cucumber mosaic virus (CMV) RNA4 in the downstream of a promoter and a gene encoding a coat protein of potato virus Y necrosis strain (PVY-N) in the downstream of the leader sequence.

The promoter used in the gene silencing vector is not particularly limited but may be any one so far as it can initiate transcription of the virus-origin coat protein. For example, 35S promoter, PAL promoter, and PAL BOX promoter may be utilized.

The leader sequence of RNA 4 in CMV is shown in Nitta et al., Japanese Journal of Phytopathology 54 : 516-522 (1988). Further, it is well known that the leader sequence of a structural protein of a plant virus generally has an effect of enhancing the expression of proteins (D.E. Sleat and T.M.A. Wilson, 1992, Plant Virus Genomes as Source of Novel functions for Genetic Engineering, In Genetic Engineering with Plant Viruses, T.M.A. Wilson and J.W. Davies ed., CRC Press, pp. 55-113).

Therefore, in the gene silencing vector , the leader sequence of the RNA4 of CMV located downstream of the promoter of GS-PVY vector is not limited, but may be any enhancer sequence so far as it can activate transcription of the expression of protein; for example, an enhancer in the 35S promoter (a region of -90 to -440) or an enhancer sequence such as TMV-Ω-sequence may be used.

It is known that the virus coat protein has a group-specific amino acid composition and is the sole virus product that has a low homology with other virus groups. PVY-T is a plant virus that belongs to Potyvirus. The gene silencing of the present invention is considered to be ascribable to protective reaction by the plant against the coat protein in the gene silencing vector of the present invention and enhancer thereof. Therefore, other gene sequences can also be used if they encode coat proteins derived from potyvirus that belongs to the same group as PVY-T and has high homologies. The examples of that include coat proteins derived from PVA, PVV, PrLV, PrMoV, TEV, TVBMV, TVMV, and TWV.

Further, in order to increase the efficiency of gene silencing, the gene sequence encoding the coat protein is not employed singly, but a plurality of same or different sequences may be arranged in tandem.

The gene silencing vector according to the present invention can be constructed based on appropriate available base vectors. It is preferable that the base vector has a replication origin that functions in the host cell in addition to the above-mentioned promoter. Furthermore, it is preferable that the base vector has a terminator and an appropriate selection marker such as drug resistance. For example, the base vector can be readily prepared by inserting the above-mentioned leader sequence of RNA4 of CMV and the sequence encoding CP of PVY-T into an appropriate available vector, by utilizing restriction enzymes according to a conventional method.

The gene silencing method is characterized in that a host plant is transformed with said gene silencing vector, wherein the vector has an additional gene that is a target of gene silencing (hereinafter, referred to as "target gene") or a gene homologous to target gene in a sense direction upstream or downstream of the above-mentioned coat protein gene of Potyvirus-origin.

The host plant in which gene silencing is to be caused by the application of the present invention is not particularly limited. Appropriate hosts include, for example, potato and tobacco.

In the present invention, the target gene construct which comprises a promoter, a sequence of the target gene and a terminator is inserted downstream of the terminator of the vector. The target gene is not particularly limited and may be any gene that exists in the host (endogenous gene) or exogenous gene or fixed one. Although the genes encoding the full-length thereof are desirable, homologous genes (for example, those encoding a part of full-length) may also be used. When a gene having a homology is used, the gene having a higher homology is more desirable; the homology is preferably 70% or more, more preferably 80% or more. Further, with regard to a number of target genes, one copy of the gene may provide the effect of the present invention sufficiently. However, to obtain a more reliable effect, a plurality of genes may be used, for example, in tandem. In this manner, incorporation of the target gene into the gene silencing vector and transformation of the host plant therewith can induce suppression of the target gene in the host.

The method of transforming a host plant is not particularly limited and an appropriate method may be selected depending on the kind of the host plant. When the host is a cell of a plant, it is preferable that *Agrobacterium tumefaciens*, which has high infectivity to plants, is first transformed with the gene construct obtained by incorporating a target gene into the above-mentioned gene silencing vector, and then, the transformant is inoculated to the host plant to effect infection. The method of transforming *Agrobacterium tumefaciens* per se is known and can be performed by, for example, a freeze-thaw method (Cited Document: G. An et al., (1988) Binary Vectors, In Plant Molecular Biology Manual A3, Kluwer Academic, Dordrecht pp. 1-19).

Accordingly, gene silencing of a target gene is induced in a high probability.

Further, individuals in which expression of a target gene is controlled at various levels (suppressed in a range of several% to 100%) can be obtained.

Still further, it is confirmed that the gene silencing of a target gene induced by the gene silencing vector is not only effective in the present generation in which transformation is performed, but also be inherited in self fertilized progeny so that the expression of target gene can be suppressed stably and consecutively.

Yet further, since the induced gene silencing of target gene is inherited in accordance with the Mendel's laws, the expression of the target gene can be suppressed at various levels not only in self fertilized progeny but also in progeny crossed with other strains and can be suppressed completely (100%). For example, as described in examples hereinbelow, about 30 individuals (about 50%) out of 60 individuals of F1 completely suppressed the expression of GFP used as a marker gene, and the remaining 30 individuals (about 50%) showed from low to medium level of suppression of GFP expression, thus showing the effect of gene silencing.

Hereinafter, the present invention will be described in more detail by way of examples. However, the examples are provided for the purpose of illustrating the present invention and should not be considered to limit the scope of the present invention described in the Claims.

### Example 1: Experiments using GFP gene as a marker (Suppression of exogenous gene in the present generation)

### <Material and Method>

### I. Preparation of vector

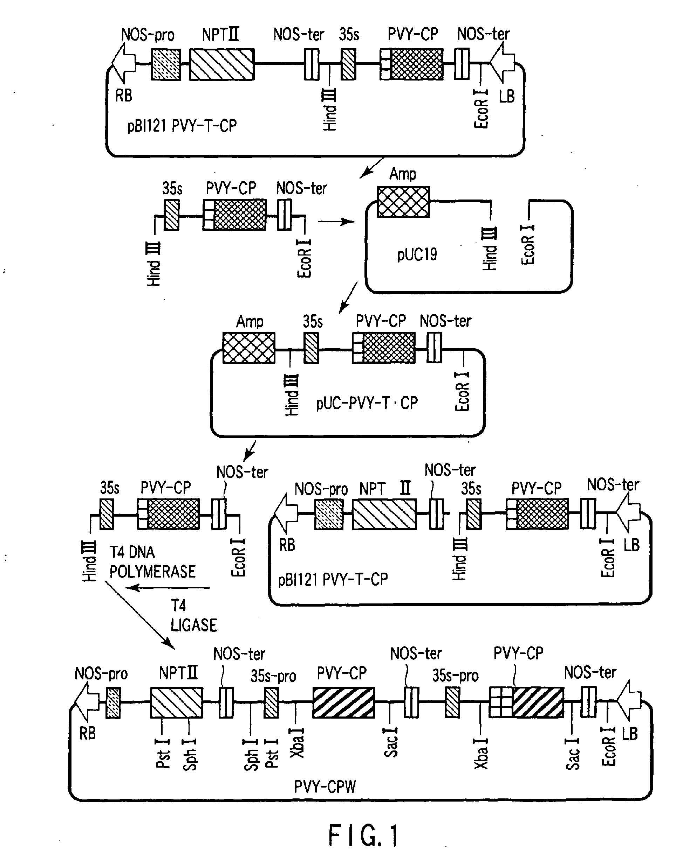

### (1) Construction of PVY-T/CPW vector: See FIG. 1

A vector pBI121PVY-T/CP (JP 6-133783 A) obtained by incorporating PVY-T/CP into a plant expression vector pBI121 (manufactured by Clontech Corporation, U.S.A.) was cleaved with restriction enzymes HindIII and EcoRI to afford a DNA fragment containing a 35S promoter, PVY-T/CP, and Nos terminator. The fragment was then introduced into the restriction enzyme sites HindIII and EcoRI of pUC19 (manufactured by Takara Shuzo Co., Ltd.) to give pUC19-PVY-T/CP. The vector was cleaved with restriction enzymes HindIII and EcoRI to recover the DNA fragment containing a 35S promoter, PVY-T/CP, and Nos terminator. Then, the fragment was blunt-ended with T4 DNA polymerase to afford a first blunt ended DNA fragment.

On the other hand, another pBI121 PVY-T/CP was cleaved with restriction enzyme HindIII and then made blunt ended with T4 DNA polymerase. The previously blunt-ended DNA fragment was ligated to the linearized pBI121 PVY-T/CP by using T4 ligase to provide PVY-CPW in which the 35S promoter, PVY-T/CP, and Nos terminator are connected in double tandem.

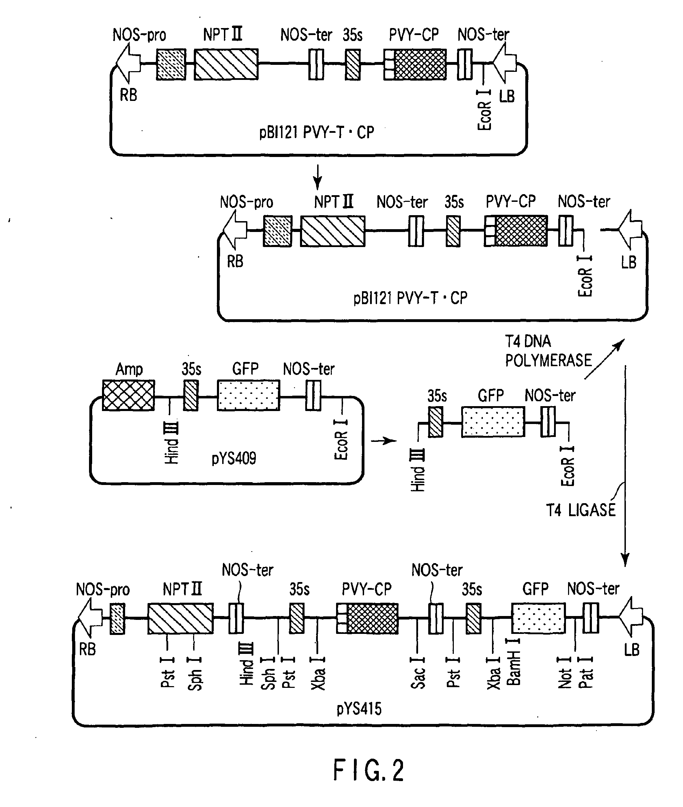

### (2) Construction of pYS415 vector: See FIG. 2

Constructed as follows was a pYS415 vector which corresponded to pBI121 PVY-T/CP vector of JP 6-133783A with a GFP gene incorporated therein.

First, pYS409 that was obtained by cloning into pUC19 (manufactured by Takara Shuzo Co., Ltd.) a 35S promoter, GFP gene, and Nos terminator was cleaved with restriction enzymes HindIII and EcoRI. DNA fragments containing the 35S promoter, GFP gene, and Nos terminator were recovered and blunt ended with T4 DNA polymerase to give blunt-ended DNA fragments. Then, pBI121PVY-T/CP was cleaved with restriction enzyme EcoRI to make them linear and then blunt-ended with T4 DNA polymerase. The previously obtained blunt-ended DNA fragment was ligated to the linearized blunt-ended pBI121 PVY-T/CP by using T4 ligase to result in vector pYS415 in which the 35S promoter, GFP gene, and Nos terminator were inserted downstream of the Nos terminator in pBI121 PVY-T/CP.

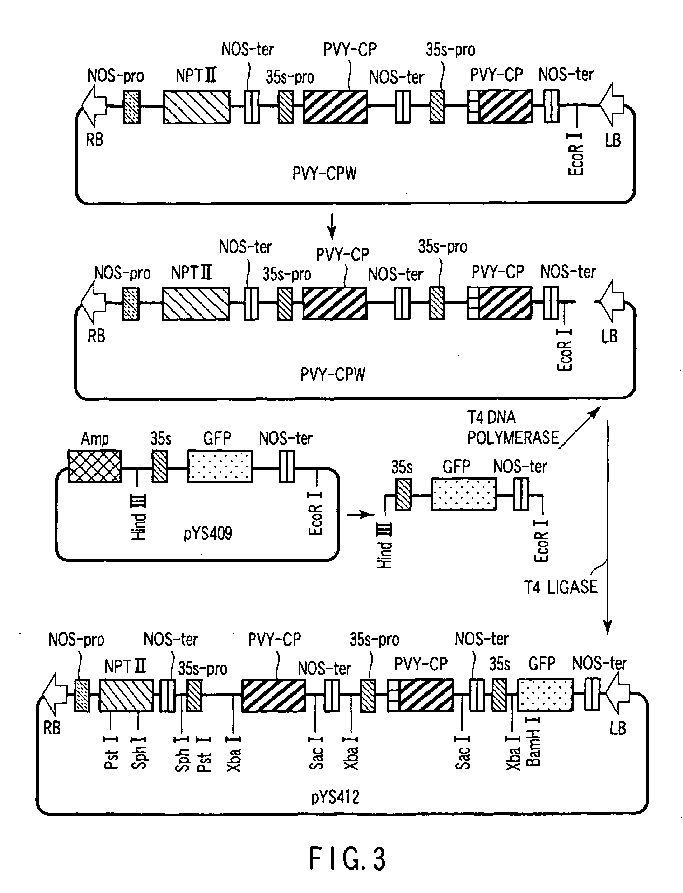

### (3) Construction of pYS412 vector: See FIG. 3

A pYS412 vector which corresponded to the PVY-T/CPW vector constructed in (1) above into which a GFP gene was incorporated was constructed as follows.

First, in the same method as in the case of pYS415 described in (2) above, pY409 that correspond pUC19 (manufactured by Takara Shuzo Co., Ltd.) having a 35S promoter, GFP gene, and Nos terminator cloned therein was cleaved with restriction enzymes HindIII and EcoRI. DNA fragments containing the 35S promoter, GFP gene, and Nos terminator were recovered and blunt ended with T4 DNA polymerase to afford blunt-ended DNA fragments. Then, the PVY-T/CPW obtained in (1) above was cleaved with restriction enzyme EcoRI to make them linear and then blunt-ended with T4 DNA polymerase the previously obtained blunt-ended DNA fragment was ligated to the linearized blunt-ended PVY-T/CPW by using T4 ligase to result in vector pYS412 in which the 35S promoter, GFP gene and Nos terminator were inserted downstream of the 3' Nos terminator in PVY-T/CPW.

### II. Transformation into Agrobacterium tumefaciens

The above-constructed plant transforming vectors pYS415 and pYS412 were separately introduced into the strain LBA4404 (Hoekema et al., Nature 303; 179-180, 1983) of *Agrobacterium tumefaciens* by a freeze-thaw method to perform transformation. Subsequently, selection by kanamycin resistance gave rise to target colonies in which either pYS415 or pYS412 was introduced into the strain LBA4404 of *Agrobacterium tumefaciens*.

### III. Transformation into Tobacco

Tobacco leaves collected from upper leaves of tobacco plant (Petit Havana SR1) cultivated for 1.5 months in a greenhouse were surface sterilized with 70% ethyl alcohol and 1% sodium hypochlorite, and washed with sterilized water, followed by preparation of leaf discs having a diameter of about 6 mm. The leaf discs together with about 10⁸ cells of *Agrobacterium tumefaciens* transformed with the vector of the present invention as described above were co-cultivated for 48 hours in a Linsmaier and Skoog's liquid medium consisting of inorganic salts and 30g/L sucrose.

Thereafter, the leaf discs were washed with sterilized water containing 250 mg/L cefotaxime to wash bacteria out, and then placed on a Linsmaier and Skoog's shoot induced medium containing inorganic salts, 0.3 mg/L 3-indoleacetic acid, 10 mg/L 2ip (6-r,r-dimethylallyl-amino) Purine, 100 mg/L kanamycin, 250 mg/L cefotaxime, and 0.9% agar. After about 1 month, the stems and leaves that showed kanamycin resistance were placed on a Linsmaier and Skoog's root induced medium containing inorganic salts, 30g/L sucrose, 100 mg/L kanamycin, 250 mg/L cefotaxime, and 0.9% agar. After the cultivation for about 1 month, the transformants that developed roots were cultivated in a greenhouse of closed system. The above-mentioned technique was performed according to the method of Komari et al. (Ther. Appl. Genet. 77, 547-552, 1989).

### IV. Analysis of transformants

### (1) Expression of GFP gene

The expression of GFP gene was analyzed by detecting fluorescence emitted by the transformant. For measuring the fluorescence, Molecular Imager FX manufactured by BIO-RAD Laboratories, Inc. was used and analysis was performed by using an image analyzing system AQUACOSMOS manufactured by Hamamatsu Photonics Co., Ltd.

### <Results>

### (1) Gene silencing of GFP gene

The fluorescence of GFP from the transformed tobacco transfected with the above-mentioned vector construct pYS415 was measured by the above-mentioned method. As a result, variety of transformed tobaccos was selected, the tobaccos varying from the individuals emitting no green fluorescence at all to individuals intensely emitting green fluorescence. Further, inoculation of PVY-T virus enabled selection of individuals that showed no disease symptom of PVY-T, i.e., immunized individuals from only those individuals which emitted no green fluorescence. The relationship between GFP expression and PVY-T resistance is shown in Table 1 below. The results indicate that in the transformed tobacco that exhibits resistance to PVY-T, the expression of GFP is suppressed by gene silencing. This indicates that the vector of the present invention suppresses the expression of the introduced GFP gene in high occurrences.

**Table 1: Relationship between GFP expression and PVY-T resistance in transformed tobacco in which GS-PVY vector + GFP gene was introduced**

| Intensity of green fluorescence | Number of individuals of transformed tobacco | Number of PVY-T resistant individuals |
|---|---|---|
| Strong | 2 | 0(0%) |
| Weak | 6 | 0(0%) |
| None | 8 | 4 (50%) |

| | | |
|---|---|---|
| Test tobacco plant: Petit Havana SR1 Test construct: pYS415 | | |

Note that the PVY-T resistance of the transformed tobacco was examined according to the assay described in the paragraph [0027] of JP 6-133783 A. That is, purified virus particles of PVY-T were inoculated to tobacco Bright Yellow No. 4 or Burley 21. After confirming the symptom of necrosis after 2 weeks, the infected leaves were sampled, diluted with PBS-T buffer (0.02M phosphate buffer) 2 to 10 times the fresh weight of the leaves, and then homogenized. The homogenate was inoculated to each transformant. The transformant subjected to the test was acclimatized with 12-cm nail and cultivated in a greenhouse of closed system controlled at about 21°C. After 2 to 3 weeks from the acclimatization, artificial inoculation was performed. The artificial inoculation was performed by coating 5 leaves of from upper to middle leaves of the test plant with a mixture of 600-mesh carborundum and a predetermined concentration of virus homogenate. After the inoculation, presence or absence of necrosis symptom was examined with time over 1 week to 2 months. Furthermore, presence or absence of virus particles was examined by using ELISA to assay degree of the resistance.

### Example 2: Experiments using GFP gene as a marker (Suppression of endogenous gene by crossing with other species)

Among the transformed tobaccos of Example 1, individuals (GBS18, GBS19) that showed resistance to PVY-T and completely suppressed expression of GFP due to gene silencing were selected. After self fertilizing the transformed tobaccos, the individuals (GBS18-13, GBS19-7, GBS20-9) showing resistance to PVY-T and exhibiting suppressed expression of GFP were selected from the resultant self fertilizing progeny (R1). Note that gene silencing was induced in an early stage for the GBS19 line while GBS18 and GBS20 lines are lines for which gene silencing was induced with aging.

Next, GBS18-13 and GBS19-7 selected as described above were crossed with transformed tobacco (GFP-7-11) obtained by introducing and fixing GFP gene into tobacco (Petit Havana SR1) so that GFP could be expressed in a high rate. F1 seeds selected were surface sterilized with 70% ethyl alcohol (for several seconds) and 1% sodium hypochlorite (5 minutes), washed with sterilized water by a conventional method, and disseminated on a Linsmaier and Skoog's medium, followed by measuring green fluorescence of GFP with time using Molecular Imager Fx manufactured by BIO-RAD Laboratories, Inc. The cultivation conditions were as follows.

Light condition: Irradiated under a white fluorescent lamp of 3,000 to 5,000 Lux for 24 hours.

Cultivation temperature: 23°C to 25°C.

Note that the self fertilizing seed and F1 hybrid seed of tobacco were harvested as follows. The self fertilizing seeds can be readily obtained by covering the tobacco inflorescence by a paper bag in order to prevent mixing of pollens. Further, F1 hybrid seeds can be obtained by emasculating individuals to be hybridized with objective individuals before crossing while taking care so that pollens from other individuals should not mix in, attaching pollens of anther that has just been cleaved to stigma of a pistil and then covering the inflorescence by an over bag. Usually, after 4 to 5 weeks from blooming, capsules turn brown and seeds ripe. Recombinant tobaccos are cultivated in a completely closed greenhouse system controlled to 20°C to 25°C, and then, self fertilized seeds and F1 hybrid seeds of tobacco were harvested.

As a result, in all the fixed F1 hybrid (GFP7-11 x SR1cont.) between the transformed tobacco (yellow) of which GFP is expressed in a high rate and non-transformed tobacco (red), expression of GFP (orange) was observed. On the contrary, in crossing with GBS18-13, GBS19-7 and GBS20-9, respectively, about 30 individuals out of 60 individuals of F1 (about 50%) showed completely suppressed expression of GFP (red), and the remaining 30 individuals (50%) showed results in which expression of GFP was suppressed at low to medium levels (orange to red). Further, examination of resistance to PVY-T indicated that all individuals in which GFP expression was completely suppressed (red individuals) showed resistance to PVY-T. On day 8 from aseptic seeding, about half of GBS19-7×GFP7-11 (F1) was completely suppressed of expression of GFP (reddening), showing such a result that gene silencing was induced as aging proceeded like the self fertilizing progenies of GBS18-13 and GBS20-9.

The above results indicate that the gene silencing effect induced in tobacco by GS-PVY vector is also maintained in the self fertilizing progeny (R1) and further succeeded in F1 generation obtained by crossing thereof with other tobacco varieties.

### Example 3: Experiments using GUS gene as a marker

### <Material and Method>

### I. Preparation of vector

A pYS436 vector, which corresponds to the PVY-T/CPW vector constructed in (1) above of Example 1 and has a β-glucuronidase (GUS) gene incorporated therein, was constructed as follows.

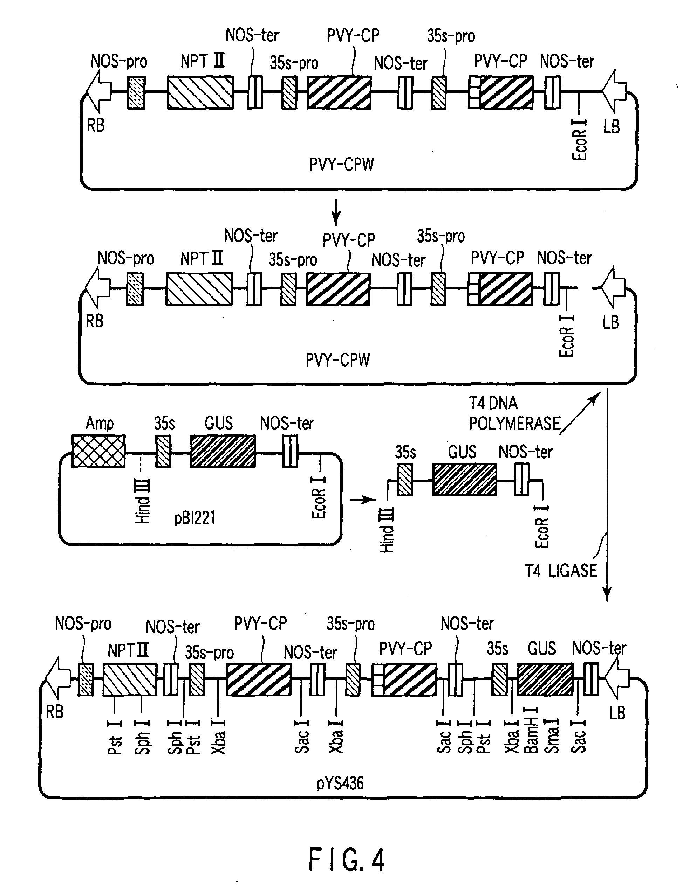

First, pBI221 which corresponds to pUC19 (manufactured by Takara Shuzo Co., Ltd.) having 35S promoter, GUS gene, and Nos terminator cloned therein was cleaved with restriction enzymes HindIII and EcoRI. DNA fragments containing the 35S promoter, GUS gene, and Nos terminator were recovered and blunt-ended with T4 DNA polymerase to obtain blunt-ended DNA fragments. Then, PVY-T/CPW was cleaved with restriction enzyme EcoRI to make them linear and then blunt-ended with T4 DNA polymerase. Ligation of the previously obtained blunt-ended DNA fragment to the linearized blunt-ended PVY-T/CPW with T4 ligase resulted in vector pYS436 in which the 35S promoter, GUS gene, and Nos terminator were inserted downstream of the 3' Nos terminator in PVY-T/CPW (see FIG. 4).

### II. Transformation into Agrobacterium tumefaciens

The above-constructed plant transforming vector pYS436 was introduced into the strain LBA4404 (Hoekema et al., Nature 303; 179-180, 1983) of *Agrobacterium tumefaciens* to perform transformation in the same method as in Example 1. Subsequently, selection by kanamycin resistance gave rise to objective colonies in which pYS436 was introduced into the strain LBA4404 of *Agrobacterium tumefaciens*.

### III. Transformation into tobacco

The obtained vector pYS436 was transformed into leaf discs of tobacco plant (Petit Havana SR1) cultivated in a greenhouse for 1.5 months, in the same method as in Example 1.

### IV. Analysis of transformants

### (1) Expression of GUS gene

First, the expression of GUS gene in *Agrobacterium tumefaciens* (LBA4404) in which GUS gene was introduced by vector pYS436 was examined. As a control, *Agrobacterium tumefaciens* (LBA4404) transformed with vector pBI121 (Clontech Corporation, U.S.A) which has a GUS gene incorporated therein was used. As a result, both *Agrobacterium tumefaciens* in which pYS436 was introduced and *Agrobacterium tumefaciens* in which pBI121 was introduced showed blue color, which indicates that in Agrobacterium, both pYS436 and pBI121 express the GUS gene.

Then, expressions of a GUS gene were examined, for transformed tobaccos into which pYS436 and pBI121 were introduced by using the above-mentioned *Agrobacterium.* As a result, the leaf discs of the transformed tobacco in which pBI121, as a control, was introduced showed blue color while the leaf discs of the transformed tobacco into which pYS436 was introduced did not show blue color. The results are summarized in Table 2.

**Table 2 GUS Expression of transformants into which pBI121 and pYS436 were introduced, respectively**

| Transformant | Number of tested individuals | Expression of GUS gene |
|---|---|---|
| pBI121 | 13 | 13 |
| pYS436 | 14 | 0 |

The above results indicate that in the transformed tobacco into which pYS436 is introduced, the expression of GUS gene is suppressed by gene silencing.

### Example 4: Experiments using a pea zinc finger transcription factor PsDof1 as a marker (suppression of endogenous transcription factor)

### <Material and Method>

### I. Preparation of vector:

First, a pYS465 vector, which corresponds to the PVY-T/CPW vector constructed in (1) above of Example 1 and has a 35S promoter, pea zinc finger transcription factor PsDof1, and Nos terminator incorporated therein, was constructed as follows.

pGEX-5X-1 having PsDof1 incorporated therein (Cited Document: Plant Biotechnology, 19(4), 251-260 (2002)) was cleaved with restriction enzymes SalI and XhoI, and a fragment containing PsDof1 gene was recovered. This was blunt-ended with T4 DNA polymerase to obtain blunt-ended DNA fragment.

Then, pCaMCN (manufactured by Pharmacia AB) was cleaved with SalI to remove CAT gene, and blunt-ended with T4 DNA polymerase. The resultant blunt-ended DNA fragment was connected to the previously obtained PsDof1 gene fragment using T4 ligase to obtain a vector pCaMCN-PsDof1 in which 35S promoter was connected upstream of the PsDof1 gene fragment and Nos terminator was connected downstream thereof.

Then, pCaMCN-PsDof1 was cleaved with XbaI and EcoRI, and then, recovered was a gene fragment in which a 35S promoter was connected upstream of the PsDof1 gene fragment and a Nos terminator was connected downstream thereof. This was blunt-ended with T4 DNA polymerase to obtain a blunt-ended DNA fragment.

Next, the pBI101.2 (Toyobo) to which GUS gene was connected was cleaved with XbaI and EcoRI to remove the GUS gene and blunt-ended with the T4 DNA polymerase. The resultant blunt-ended DNA fragment was connected to the previously obtained gene fragment in which the 35S promoter was connected upstream of the PsDof1 gene fragment and Nos terminator was connected downstream thereof by using T4 ligase, thereby obtaining a vector pBI101.2-PsDof1.

Then, pBI101.2-PsDof1 was cleaved with HindIII and EcoRI to recover the gene fragment in which the 35S promoter was connected upstream of the PsDof1 gene fragment and Nos terminator was connected downstream thereof, and this fragment was blunt-ended with T4 DNA polymerase to obtain a blunt-ended DNA fragment.

Next, the PVY-T/CPW obtained in (1) of Example 1 was cleaved with restriction enzyme EcoRI and blunt-ended with T4 DNA polymerase. The previously obtained blunt-ended DNA fragment was connected to the linearized blunt-ended PVY-T/CPW with T4 ligase, thereby resulting in vector pYS465 in which the 35S promoter, PsDof1 gene, and Nos terminator were inserted downstream of the 3' Nos terminator in PVY-T/CPW.

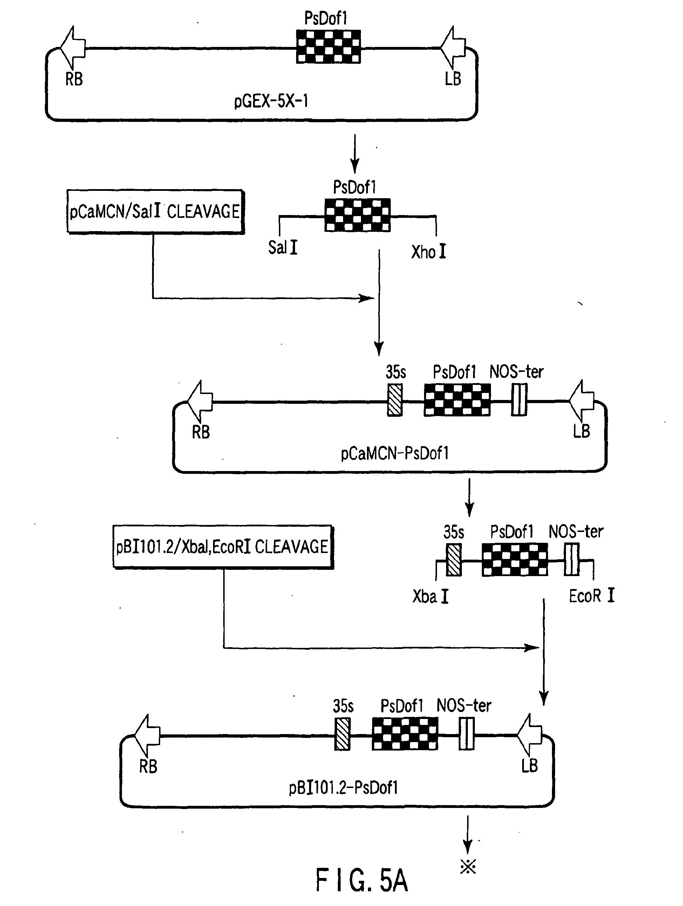

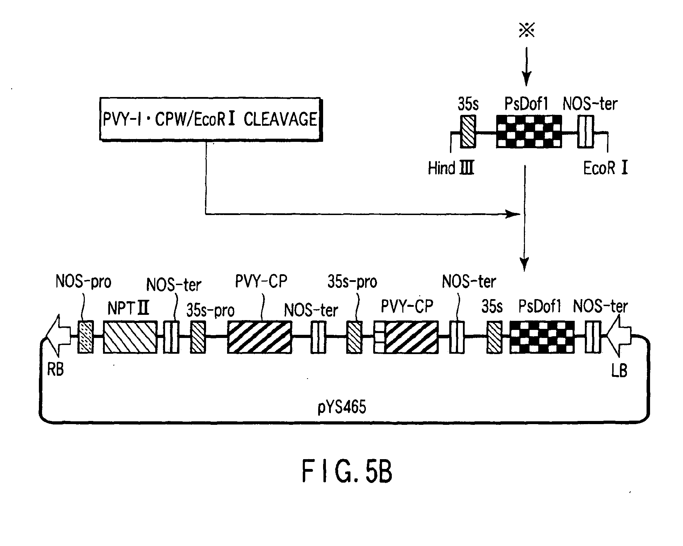

Further, constructed was a pYS466 vector in which PAL promoter, pea zinc finger transcription factor PsDof1, and Nos terminator were incorporated into PVY-T/CPW vector (see FIGS. 5A and 5B).

pUC18 having BOX 5 repeater promoter and CAT gene incorporated therein (JP 2000-245463 A) was cleaved with restriction enzymes HindIII and BamH1 to recover the PAL promoter DNA fragment.

Next, pBI101.2-PsDofl was cleaved with HindIII and BamH1 to remove the 35S promoter, and the resultant fragment was connected to the previously obtained PAL promoter with T4 ligase, thereby obtaining a pBI101.2-PAL-PsDof1 vector. Furthermore, the pBI101.2-PAL-PsDof1 was cleaved with HindIII and EcoRI to recover a fragment that contained the PAL promoter, PsDof1 gene, and Nos terminator, and the resultant fragment was blunt-ended with T4 DNA polymerase.

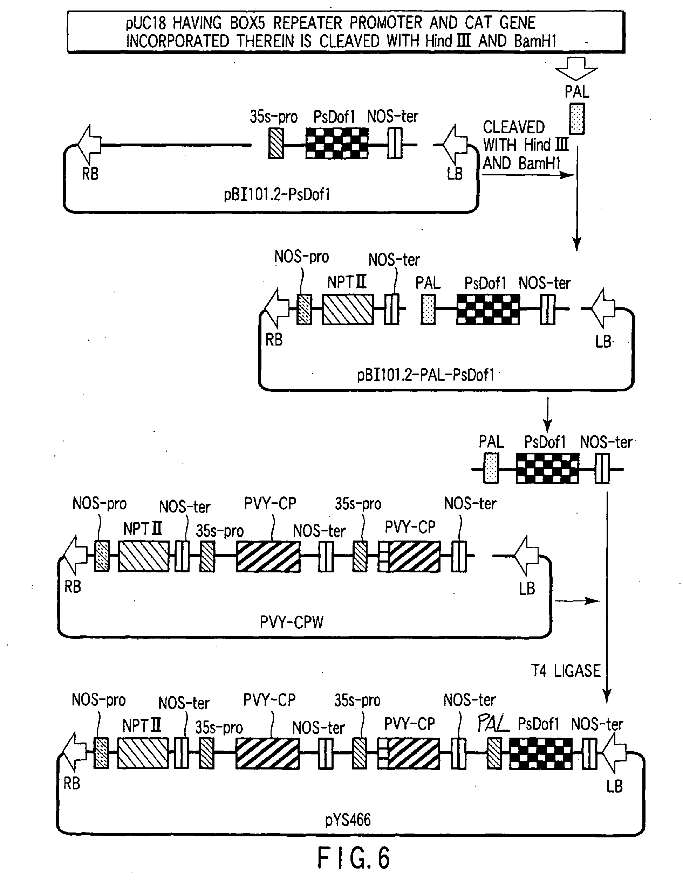

Then, PVY-T/CPW was cleaved with restriction enzyme EcoRI and blunt-ended with T4 DNA polymerase. The linearized blunt-ended PVY-T/CPW was connected to the previously obtained blunt-ended DNA fragment with T4 ligase, thereby giving rise to a vector pYS466 in which the PAL promoter, PsDof1 gene, and Nos terminator were inserted downstream of 3' Nos terminator in PVY-T/CPW (see FIG. 6).

### II. Transformation into Agrobacterium tumefaciens

The above-constructed plant transforming vectors pYS465 and pYS466 were introduced into the strain LBA4404 (Hoekema et al., Nature 303; 179-180, 1983) of *Agrobacterium tumefaciens* to perform transformation in the same method as Example 1. Subsequently, selection by kanamycin resistance was performed, thereby obtaining target colonies in which either pYS465 or pYS466 was introduced into the strain LBA4404 of *Agrobacterium tumefaciens*.

### III. Transformation into tobacco

The obtained vectors pYS465 and pYS466 were transformed into leaf discs of tobacco plant (Petit Havana SR1) cultivated in a greenhouse for 1.5 months, in the same method as in Example 1.

### IV. Analysis of transformants

### (1) Gene silencing of transcription factor gene

Observation of the morphological changes of the tobacco transformed with the vector pYS465 or pYS466 indicated that the pYS465 transformants showed a tendency of delayed initial growth. On the other hand, some of the pYS466 transformants showed morphological abnormality such as formation of wrinkles on the surface of leaves and shrinking of the whole leaf. This is considered to be attributable to the occurrence of gene silencing of a transcription factor having a homology to the transcription factor PsDof1.

As described above in detail, according to the present invention, remarkable effects such as achievement of gene silencing of a specific target gene in a host can be attained.

## Claims

1. Use of a gene silencing vector for silencing a target gene in a host plant, said gene silencing vector comprising:
a recombinant vector which includes
a promoter,
a leader sequence of RNA4 of cucumber mosaic virus downstream of the promoter,
a gene encoding a coat protein of a potato virus y necrosis strain (PVY-N) which is downstream of the leader sequence, and
a terminator that is downstream of the gene,
wherein in order to cause gene silencing of the specific target gene in a host plant, a gene construct which comprises a promoter, a sequence of the target gene and a terminator is inserted downstream of the terminator of the vector.

2. The use according to claim 1, wherein said promoter is selected from the group consisting of 35S-promoter, PAL-promoter and PAL BOX promoter.

3. The use according to claim 1, wherein a plurality of the coat proteins is incorporated to suppress expression of the target gene more strongly.

4. The use according to claim 1, wherein expression of the target gene is suppressed, and the suppression is stably maintained not only in present generation of transformants but also in self fertilizing progeny.

5. The use according to claim 4, wherein said transformant is further crossed with another plant to afford a hybrid generation, and the gene silencing of the target gene is maintained in the hybrid generation.

6. The use according to claim 1, wherein the host plant is tobacco.

## Patentansprüche

1. Verwendung eines Gen-Silencing-Vektors zum Stilllegen (Silencing) eines Zielgens in einer Wirtspflanze, wobei der Gen-Silencing-Vektor umfasst:
einen rekombinanten Vektor, der
einen Promotor,
eine Leader-Sequenz der RNA4 des Gurkenmosaikvirus stromabwärts des Promotors, ein Gen, das fiir ein Hüllprotein eines Nekrosestamms des Kartoffelvirus Y (PVY-N) codiert und sich stromabwärts der Leader-Sequenz befindet, und
einen Terminator, der sich stromabwärts des Gens befindet,
einschließt,
wobei zum Erreichen des Stilllegens des spezifischen Zielgens in einer Wirtspflanze ein Genkonstrukt, das einen Promotor, eine Sequenz des Zielgens und einen Terminator umfasst, stromabwärts des Terminators des Vektors insertiert wird.

2. Verwendung nach Anspruch 1, wobei der Promotor ausgewählt ist aus der Gruppe, bestehend aus 35S-Promotor, PAL-Promotor und PAL-BOX-Promotor.

3. Verwendung nach Anspruch 1, wobei eine Vielzahl von Hüllproteinen eingebaut wird, um die Expression des Zielgens noch stärker zu unterdrücken.

4. Verwendung nach Anspruch 1, wobei die Expression des Zielgens unterdrückt wird und die Unterdrückung nicht nur in der aktuellen Generation der Transformanten, sondern auch in der sich selbstbefruchtenden Nachkommenschaft stabil aufrechterhalten wird.

5. Verwendung nach Anspruch 4, wobei die Transformante weiter mit einer anderen Pflanze gekreuzt wird, um eine Hybrid-Generation zu erzeugen und wobei die Gen-Stilllegung des Zielgens in der Hybrid-Generation beibehalten wird.

6. Verwendung nach Anspruch 1, wobei die Wirtspflanze Tabak ist.

## Revendications

1. Utilisation d'un vecteur d'inactivation génique pour inactiver un gène cible dans une plante hôte, ledit vecteur d'inactivation génique comprenant :
un vecteur recombiné qui comprend
un promoteur,
une séquence leader de l'ARN 4 du virus de la mosaïque du concombre en aval du promoteur,
un gène codant une protéine d'enveloppe d'une souche de nécrose du virus Y de la pomme de terre qui est en aval de la séquence leader, et
un terminateur qui est en aval du gène,
où, pour provoquer l'inactivation génique du gène cible spécifique dans une plante hôte, on insère un montage génique qui comprend un promoteur, une séquence du gène cible et un terminateur en aval du terminateur du vecteur.

2. Utilisation selon la revendication 1, où ledit promoteur est sélectionné dans le groupe constitué du promoteur 35S, du promoteur PAL et du promoteur PAL BOX.

3. Utilisation selon la revendication 1, où une pluralité de protéines d'enveloppe est incorporée pour supprimer plus fortement l'expression du gène cible.

4. Utilisation selon la revendication 1, où l'expression du gène cible est supprimée et la suppression est conservée de façon stable non seulement dans la génération présente de transformants mais également dans la descendance autofécondante.

5. Utilisation selon la revendication 4, où ledit transformant est croisé en outre avec une autre plante pour former une génération hybride et l'inactivation génique du gène cible est conservée dans la génération hybride

6. Utilisation selon la revendication 1, où la plante hôte est le tabac.
